(19) Europäisches Patentamt
European Patent Office
Office européen des brevets

(11) **EP 1 752 151 A1**

(12) **EUROPEAN PATENT APPLICATION**

(43) Date of publication:
**14.02.2007 Bulletin 2007/07**

(21) Application number: **06254034.9**

(22) Date of filing: **01.08.2006**

(51) Int Cl.:
*A61K 31/56* (2006.01)    *C07J 9/00* (2006.01)
*A23L 1/30* (2006.01)    *A23L 2/00* (2006.01)
*A23L 2/02* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.08.2005 US 194176**

(71) Applicant: **TROPICANA PRODUCTS, INC.**
**Bradenton, FL 34208 (US)**

(72) Inventors:
• **Hitchcock, Bryan**
**Vemon Hills,**
**IL 60061 (US)**

• **Schroen, Jeff**
**Cary,**
**IL 60013 (US)**
• **Rivera, Ted**
**Algonquin,**
**IL 60102 (US)**
• **Shields, Nicholas Colin**
**Crystal Lake,**
**IL 60014 (US)**
• **Lam, David**
**75017 Paris (FR)**
• **Christiaens, Sabrina**
**3840 Borgloon (BE)**

(74) Representative: **MacLean, Martin Robert et al**
**Mathys & Squire**
**120 Holborn**
**London EC1N 2SQ (GB)**

(54) **Sterol fortified beverages**

(57) A fortified beverage is provided that comprises a sterol ester, stanol ester, or combinations thereof; and an aqueous medium. Methods of making such beverage are also provided.

EP 1 752 151 A1

**Description**

**Field of the Invention**

The present invention relates to a sterol fortified beverage which provides a medium to deliver a cholesterol reducing ester and methods for the manufacture thereof. The beverage comprised of a plant sterol ester or a plant stanol ester substantially reduces cholesterol in humans.

**Background of the Invention**

It has been known from various studies that plant sterols and plant stanois provide benefits to consumers by reducing low-density lipoprotein (LDL) cholesterol levels. However, these benefits are difficult to achieve because it is difficult to incorporate plant sterols into human food products.

Plant sterols can be derived from vegetable oils or tall oil. Common sources of sterols from vegetable oils include, but are not limited to, coconut oil, corn oil, cotton oil, olive oil, palm oil, peanut oil, rapeseed oil, canola oil, safflower oil, linseed oil, cotton seed oil, soybean oil, sunflower oil, walnut oil, and avocado oil. Additionally, sterols can be derived from tall oil. Tall oil is derived from the wood of coniferous plants. Plant stanois are saturated or hydrogenated sterols. These sterols and stanois are usually free sterols and free stanols.

A need exists for achieving the benefits that sterols and stanois can provide and for making food products that provide such benefits, without the use of added emulsifiers.

**Summary of the Invention**

In accordance with one aspect of the present invention, a beverage fortified with sterol esters, stanol esters, or combinations thereof is provided. The beverage composition typically contains plant-derived sterol esters, stanol esters, or combinations thereof in an amount as desired and preferably in an amount sufficient to reduce cholesterol in humans.

Another advantage of the present invention is that the addition of sterol or stanol esters does not affect the physical properties, such as viscosity and mouth feel, of beverages.

The beverage comprises an aqueous medium; a cholesterol reducing ester selected from the group consisting of sterol esters, stanol esters, and mixtures thereof. The sterol or stanol portion of the molecule is present in the beverage in a concentration on a molecular weight basis of the sterol or stanol portion of the ester of from about 0.8 grams/liter to about 50 grams/liter of beverage, preferably from about 1.6 grams/liter to about 12.5 grams/liter of beverage, more preferably from about 3.2 grams/liter to about 7 grams/liter of beverage, and the cholesterol reducing ester is uniformly dispersed in the aqueous medium.

The aqueous medium can be selected from juices, teas, coffees, dairy drinks, nonalcoholic drinks, carbonated drinks, noncarbonated drinks, and mixtures thereof.

The juice can be a fruit juice selected from orange, grapefruit, lime, lemon, pineapple, mango, banana, peach, grape, kiwi, cherry, watermelon, apple, strawberry, raspberry, cranberry, blueberry, blackberry, plum, passion fruit, tangerine, pomegranate, mandarin, pear, plum, mirabelle, honeydew, cantaloupe, papaya, tomato, guava, in addition to other fruits known in the art; a vegetable juice selected from celery, beets, parsley, lettuce, spinach, carrot, in addition to other vegetables known in the art; and mixtures thereof.

In accordance with another aspect of the present invention, a method of producing a beverage fortified with sterol esters, stanol esters, or combinations thereof is provided. The method comprises blending a cholesterol reducing ester selected from sterol esters, stanol esters, and mixtures thereof, and an aqueous liquid beverage component to form a mixture; a first homogenizing of the mixture to form a resulting beverage; after the first homogenizing, optionally heating said resulting beverage to a pasteurization temperature; cooling the resulting beverage to a temperature less than the melting point of the cholesterol reducing ester present in the beverage to form a cooled beverage. The sterol or stanol portion of the molecule is present in the beverage in a concentration of from about 0.8 grams/liter to about 200 grams/liter of beverage, preferably from about 1.6 grams/liter to about 12.5 grams/liter of beverage, and more preferably from about 3.2 grams/liter to about 7 grams/liter of beverage.

The first homogenizing can be performed in the absence of an added emulsifier in the beverage composition, and the homogenizing can be performed at a pressure from about 500 psi to about 9000 psi, preferably from about 750 psi to about 5000 psi, and more preferably from about 2000 psi to about 3000 psi. The resulting beverage is characterized by a uniform dispersion of the cholesterol reducing ester in the aqueous medium.

The melting point of the cholesterol reducing ester is typically from about 35°C to about 80°C.

The pasteurization temperature can be from about 70°C to about 130°C, preferably from about 85°C to about 100°C.

The cooling can be carried out at a temperature below the melting point of the cholesterol reducing ester, typically less than about 35°C, preferably less than about 4°C.

In another embodiment, cholesterol reducing ester can be in semi-solid form. In such case, prior to the blending, the cholesterol reducing ester can first be heated to a temperature above its melting point to form a liquid, and the aqueous liquid beverage component can be preheated to a temperature above the melting point of the cholesterol reducing ester.

In another embodiment, the cholesterol reducing ester can be in liquid form. In such case, prior to the blending, the aqueous liquid beverage component can be preheated to a temperature above the melting point of the cholesterol reducing ester, typically from about 35°C to about 80°C, preferably from about 55°C to about 70°C.

In one embodiment, the cholesterol reducing ester can be in powder form. In such case, the blending can be carried out at a temperature below the melting point of the cholesterol reducing ester, typically less than about 35°C, preferably less than about 4°C.

In another embodiment, the blending can be done at high concentrations of the cholesterol reducing ester to form a concentrated mixture. In such case, the method further includes diluting the mixture with an aqueous medium to a concentration of the sterol or stanol portion of the molecule of from about 0.8 grams/liter to about 50 grams/liter, preferably from about 1.6 grams/liter to about 12.5 grams/liter of beverage, and more preferably from about 3.2 grams/liter to about 7 grams/liter of beverage. The diluting can be carried out at any temperature, preferably less than about 35°C, more preferably less than about 4°C.

The method further comprises a second homogenizing of the resulting beverage to provide a uniform dispersion of the cholesterol reducing ester in the aqueous medium; heating the homogenized resulting beverage to the pasteurization temperature for a pasteurization time less than about 240 seconds, preferably less than 120 seconds, and more preferably less than 60 seconds; and cooling the homogenized resulting beverage to a temperature less than the melting point of the cholesterol reducing ester present in the beverage to form a finished beverage.

In one embodiment, the method further comprises heating the resulting beverage prior to the second homogenizing to a temperature above the melting point of the cholesterol reducing ester, typically from about 35°C to about 80°C, preferably from about 55°C to about 70°C.

In accordance with another aspect of the present invention, a system for producing a beverage fortified with sterol esters, stanol esters, or combinations thereof is provided. The apparatus comprises a source of a cholesterol reducing ester selected from the group consisting of sterol esters, stanol esters, and mixtures thereof; a source of an aqueous liquid beverage component; a mixing device for blending the cholesterol reducing ester with the aqueous liquid beverage component to form a resulting mixture; a homogenizer in fluid connection with the mixing device for homogenizing the resulting mixture to form a resulting beverage characterized by a uniform dispersion of the cholesterol reducing ester, a pasteurization device for pasteurizing the resulting beverage in fluid connection with said homogenizer to produce a pasteurized beverage; and a chilling device to cool the pasteurized beverage to a temperature below the melting point of the cholesterol reducing ester present in the beverage.

## Brief Description of the Drawings

Figure 1 is a schematic design illustrating a system in accordance with the present invention for making a sterol fortified beverage.

Figure 2 is a schematic design illustrating another system in accordance with the present invention for making a sterol fortified beverage.

## Detailed Description of the Invention

In accordance with one aspect of the present invention, a beverage fortified with a sterol ester, stanol ester, or combinations thereof is provided. The beverage composition typically can contain plant-derived sterol esters, stanol esters, or combinations thereof in an amount sufficient to reduce blood cholesterol levels.

The sterol fortified beverage comprises a cholesterol reducing ester selected from the group consisting of sterol esters, stanol esters, and combinations thereof, and an aqueous medium. The sterol or stanol portion of the molecule is present in the beverage in a concentration of from about 0.8 grams/liter to about 50 grams/liter of beverage, preferably from about 1.6 grams/liter to about 12.5 grams/liter of the beverage, and more preferably from about 3.2 grams/liter to about 7 grams/liter of the beverage; and the cholesterol reducing ester is uniformly dispersed in the aqueous medium.

Stanols in the free and esterified forms and esterified sterols are not as readily available from natural sources as the free sterols. Thus, the free sterols must be hydrogenated to produce free stanols; and the free sterols and stanols must be esterified to produce sterol esters and stanol esters. The esterification of the free sterols and stanols comprise reacting the free sterol or stanol with a fatty acid ester which can be a mixture of a vegetable oil, such as rapeseed oil, and methyl ester. A process for esterifying free stanols and a composition of esterified stanols are set forth in U.S. Patent Nos. 5,958,913 and 6,174,560. These references are incorporated by reference in this written description in their entirety, including any references cited therein. In addition, suitable cholesterol reducing sterols and stanols in esterified forms are commercially available from Raisio Benecol and McNeil Nutritionals, under the trade name Benecol®, from Archer Daniels Midland under the trade name CardioAid®, from Cognis under the trade name VegaPure®, from MultiBene Group under the trade name MultiBene® and others as known in the art.

The cholesterol reducing ester of the present invention is available in various physical forms at ambient temperature. Examples of the cholesterol reducing ester in its various forms include, but are not limited to, semi-solid esters, liquid esters, powder esters, and combinations thereof.

The term "sterol or stanol portion of the molecule" means the sterol or stanol part of a sterol ester or stanol ester molecule respectively exclusive of the fatty acid ester part. Figure A below shows the fatty ester part and the stanol portion of a sitostanol ester molecule. The molecule below is intended only for illustrative purposes and is not intended to limit the present invention to the same. Other stanol esters such as campestanol ester and others known to those skilled in the art can be used with the present invention.

**Figure A. Sitotanol Ester Molecule**

The amount of sterol or stanol portion of the ester present in a beverage can be readily calculated, as is well known to those skilled in the art. For example, if the ester has a molecular weight $x$ and the sterol or stanol portion has a molecular weight $y$ and the fatty acid ester portion has a molecular weight $z$, then if 25 grams per liter of the sterol or stanol ester are present in a beverage, the concentration of the sterol or stanol portion will be

$$\frac{y_{\text{(sterol or stanol portion molecular weight)}}}{x_{\text{(total sterol or stanol ester molecular weight)}}} \times 25 \text{ grams/liter},$$

which in this case equals

$$\frac{y_{\text{(sterol or stanol portion molecular weight)}}}{y_{\text{(sterol or stanol portion molecular weight)}} + z_{\text{(fatty acid ester portion molecular weight)}}} \times 25 \text{ grams/liter}.$$

The sterol and stanol ester molecule typically has a fatty-acid-ester aterol or stanol portion of the molecule molecular weight ratio of from about 0.25:1 to about 1.2:1, preferably from about 0.5:1 to about 1:1.

Typically, the concentration of the sterol or stanol portion of the molecule present in the beverage will be an amount sufficient to reduce cholesterol in humans. Typically, the amount of the sterol or stanol portion of the cholesterol reducing ester molecule present in the beverage is from about 0.8 grams/liter to about 50 grams/liter of the beverage, preferably from about 1.6 grams/liter to about 12.5 grams/liter of the beverage, and more preferably from about 3.2 grams/liter to about 7 grams/liter of the beverage. These concentrations can result in an amount from about 0.4 grams to about 3.0 grams of sterol or stanol portion of the ester molecule per single-serving size of 8 fluid ounces.

The aqueous medium of the present invention includes, but is not limited to, juices, teas, coffees, dairy drinks, nonalcoholic drinks, carbonated drinks, noncarbonated drinks, or combinations thereof. In a preferred embodiment, the aqueous medium is a fruit juice, preferably orange juice. Suitable juices can be used as desired. Other examples of fruit juices that can be used in the present invention include, but are not limited to, orange, grapefruit, lime, lemon, pineapple, mango, banana, peach, grape, kiwi, cherry, watermelon, apple, strawberry, raspberry, cranberry, blueberry, blackberry, plum, passion fruit, tangerine, pomegranate, mandarin, pear, plum, mirabelle, honeydew, cantaloupe, papaya, tomato, guava, in addition to other fruits known in the art, and combinations thereof. Other juices like vegetable juice can be used in the present invention. Examples of suitable vegetable juices include, but are not limited to, celery, beets, parsley, lettuce, spinach, carrot, in addition to other vegetables known in the art, and mixtures thereof.

Those skilled in the art will recognize that certain beverages, such as fruit juices, can experience some degree of natural separation, for example pulp settling. In the present invention, the cholesterol reducing ester is uniformly dispersed. The term "uniformly dispersed" and variations thereof such as "uniform dispersion" means cholesterol reducing ester dispersions in beverages that do not separate or otherwise cause neck ringing or floccing in the containers in addition to the separation that can naturally occur in certain beverages.

In accordance with another aspect of the present invention, a method of producing a beverage fortified with sterol esters, stanol esters, or combinations thereof is provided. The method comprises blending a cholesterol reducing ester and an aqueous liquid component to form a mixture. The cholesterol reducing ester is present in an amount such that the sterol or stanol portion of the molecule is present in the beverage in a concentration on a molecular weight basis of the sterol or stanol ester of from about 0.8 grams/liter to about 200 grams/liter of beverage and the cholesterol reducing ester is uniformly dispersed in the aqueous medium.

In one embodiment, the cholesterol reducing ester is in a semi-solid physical state at ambient temperature. Thus, in accordance with the method of the present invention and prior to blending, the cholesterol reducing ester is first heated to a temperature above its melting temperature to form a liquid and the aqueous medium is heated to a temperature above the melting point of the cholesterol reducing ester. To those skilled in the art, the melting temperature of the cholesterol reducing ester will be apparent from its composition. In the present invention, the melting temperature is typically from about 35°C to about 80°C, preferably from about 55°C to about 70°C.

In another embodiment, the cholesterol reducing ester is in a liquid physical state at ambient temperature. Thus, in accordance with the method of the present invention and prior to blending, the aqueous medium is first heated to a temperature above the melting point of the cholesterol reducing ester.

In yet another embodiment, the cholesterol reducing ester is in a powder physical state at ambient temperature. Thus, in accordance with the method of the present invention, the blending may be done at a temperature above or below the melting point of the cholesterol reducing ester, more preferably less than about 35°C, even more preferably less than about 4°C. The method further comprises dispersing said cholesterol reducing ester using standard mixing techniques known to those skilled in the art.

When the cholesterol reducing ester is in the semi-solid state or the liquid state, it is necessary to blend the cholesterol reducing ester and the aqueous medium at a temperature above the melting point of the ester. This is required to prevent the cholesterol reducing ester from solidifying prior to the first homogenizing. However, when the cholesterol reducing ester is in powder form, the blending can be done at a temperature above the melting point of the cholesterol reducing ester but this is not required.

In one embodiment, the amount of the cholesterol reducing ester blended with the aqueous medium is sufficient to provide a desired concentration of the sterol or stanol portion of the molecule in the mixture. In the present invention, the desired sterol or stanol portion of the molecule concentration is from about 0.8 grams/liter to about 50 grams/liter of the mixture, preferably from about 1.6 grams/liter to about 12.5 grams/liter of the mixture, and more preferably from about 3.2 grams/liter to about 7 grams/liter of the mixture.

In a further embodiment, the amount of cholesterol reducing ester blended with the aqueous medium is sufficient to provide a concentrated mixture of the cholesterol reducing ester several magnitudes greater than the desired concentration. For example, in a concentration of four times (4x) the desired concentration in the present invention, the sterol or stanol portion of the molecule concentration is from about 3.2 grams/liter to about 200 grams/liter of the mixture, preferably from about 6.4 grams/liter to about 50 grams/liter of the mixture, and more preferably from about 12.8 grams/liter to about 28 grams/liter of the mixture.

The method further comprises homogenizing the mixture at a pressure from about 500 psi to about 9000 psi, preferably from about 750 psi to about 5000 psi, and more preferably from about 2000 psi to about 3000 psi, to form a resulting beverage characterized by a uniform dispersion of the cholesterol reducing ester in the resulting beverage. Furthermore, the homogenizing reduces the particle size of the cholesterol reducing ester in the beverage to provide a desirable mouth feel, i.e. no grittiness is noticeable.

In the present invention, the homogenization is performed in the absence of added emulsifiers in the beverage or the cholesterol reducing ester. Those skilled in the art will appreciate that an aqueous medium may contain an emulsifier to stabilize a dispersion, other than the cholesterol reducing ester dispersion, in the aqueous medium. However, in the present invention no emulsifier is used to stabilize the dispersed cholesterol reducing ester in the aqueous medium.

In addition, the cholesterol reducing ester used in the present invention is free of emulsifiers. Added emulsifiers, as used in the present invention, refers to emulsifiers used to otherwise stabilize the dispersed cholesterol reducing ester in the aqueous medium or to emulsifiers used as an additional component of the cholesterol reducing ester.

In the present invention, homogenizing techniques available to uniformly disperse the cholesterol reducing ester include, but are not limited to, homogenizing, shearing, vortexing, sonicating, or other mixing techniques known to those skilled in the art.

In one embodiment, said homogenizing is carried out at a temperature less than about 35°C, preferably less than about 4°C.

In another embodiment, said homogenizing is carried out a temperature from about 70°C to about 130°C, preferably from about 85°C to about 100°C.

Those skilled in the art will appreciate that not all aqueous mediums, for example carbonated cola drinks, require pasteurization to form a finished beverage. If pasteurization is desired, the pasteurization can be done using techniques known to those skilled in the art. To a skilled artisan it will be apparent that as the pasteurization temperature increases, the holding time of the beverage at the pasteurization temperature decreases. Conversely, as the pasteurization temperature decreases, the holding time of the beverage at the pasteurization temperature increases. Further, it will be apparent to those skilled in the art that the beverage should be held at the pasteurization temperature for less than about 240 seconds, preferably less than about 120 seconds, and more preferably less than about 60 seconds, to substantially reduce unwanted microorganisms and to substantially deactivate unwanted enzymes in the beverage. This process is known in the art as reducing the microbial load or variations thereof such as microbial load reduction.

In the present invention, after said homogenizing, the method further comprises optionally heating the resulting beverage to a pasteurization temperature from about 70°C to about 130°C, preferably from about 85°C to about 100°C and holding the resulting beverage at the pasteurization temperature for less than about 240 seconds, preferably less than about 120 seconds, and more preferably less than about 60 seconds.

In another embodiment, the method further comprises cooling the resulting beverage to a temperature less than about 35°C, preferably less than about 4°C.

When the mixture is a concentrated mixture of the cholesterol reducing ester, the method further comprises diluting the resulting beverage with an aqueous medium to a sterol or stanol portion of the molecule concentration of from about 0.8 grams/liter to about 50 grams/liter of the beverage, preferably from about 1.6 grams/liter to about 12.5 grams/liter of the beverage, and more preferably from about 3.2 grams/liter to about 7 grams/liter of the beverage at a temperature less than about 35°C, preferably less than about 4°C.

The method further comprises preheating the resulting beverage to a temperature above the melting point of the esters present in the beverage wherein said temperature is from about 35°C to about 80°C, preferably from about 55°C to about 70°C; homogenizing the resulting beverage at a pressure from about 500 psi to about 9000 psi, preferably from about 750 psi to about 5000 psi, and more preferably from about 2000 psi to about 3000 psi, to form a finished beverage characterized by a uniform dispersion of the esters in the finished beverage; heating the finished beverage to the pasteurization temperature for less than about 240 seconds, preferably less than about 120 seconds, more preferably less than about 60 seconds; and cooling the finished beverage to a temperature below the melting point of the cholesterol reducing ester, preferably less than about 35°C, more preferably less than about 4°C.

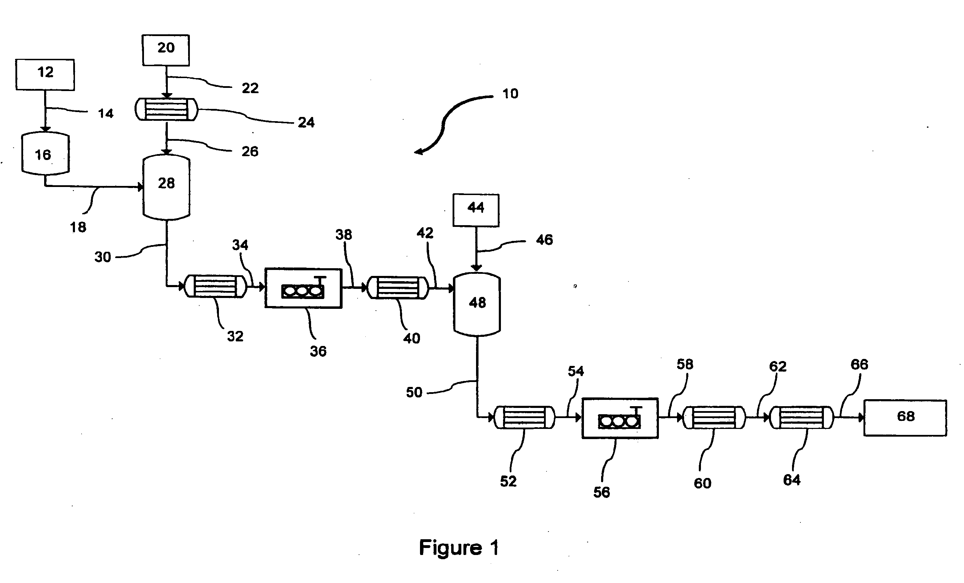

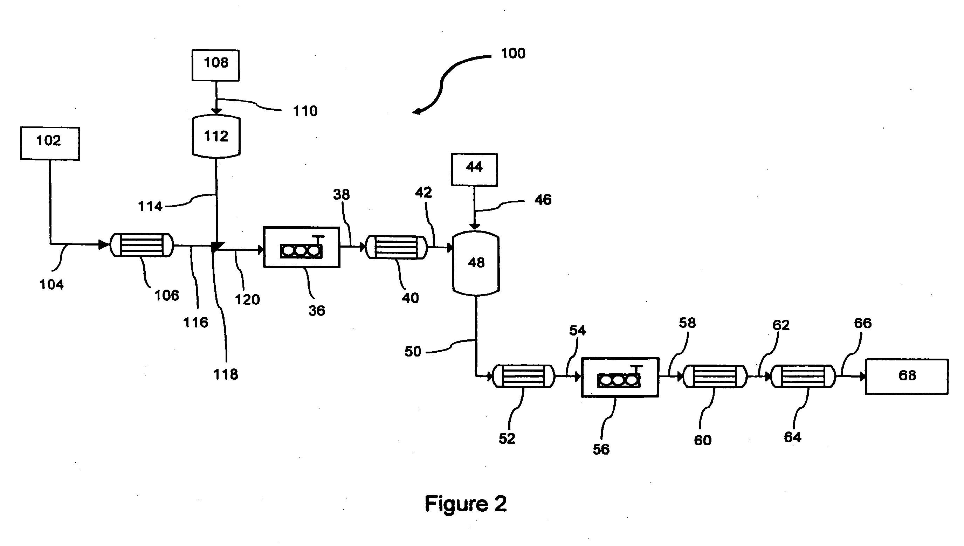

Referring now to the drawings, Figures 1 and 2 are provided to illustrate embodiments of the present invention and not for the purpose of limiting the same. Figure 1 is a schematic design illustrating a system in accordance with the present invention for making a sterol fortified beverage. A system 10 is provided to manufacture a sterol or stanol ester fortified beverage. A suitable source 12 of a semi-solid cholesterol reducing ester is supplied via a conduit 14 into a melting tank 16 where the cholesterol reducing ester is melted to form a liquefied cholesterol reducing ester. A stream 18 of the liquefied cholesterol reducing ester is then pumped into a blending tank 28 in fluid connection with melting tank 16. A suitable source 20 of an aqueous liquid beverage component is supplied via a conduit 22 into a heat exchanger 24 to heat the aqueous liquid beverage component to a temperature above the melting point of the cholesterol reducing ester. The heated aqueous liquid beverage component is pumped to blending tank 28 where the liquefied cholesterol reducing ester is blended with the heated aqueous liquid beverage component to form a concentrated mixture. A stream 30 of the concentrated mixture is then pumped into a heat exchanger 32 to heat the concentrated mixture to a temperature above the melting point of the cholesterol reducing ester. A stream 34 of the heated concentrated mixture is then pumped into a homogenizer 36 to form a uniformly dispersed concentrated mixture. A stream 38 of the concentrated mixture is then pumped into a chiller 40 to cool the concentrated mixture to a temperature below the melting point of the cholesterol reducing ester, preferably less than 4°C. A stream 42 of the concentrated mixture is then pumped into a blending tank 48. A suitable source 44 of an aqueous liquid component is supplied via a conduit 46 into blending tank 48. The concentrated mixture is blended with the aqueous liquid component in blending tank 48 to dilute the concentrated mixture to a desired concentration of the cholesterol reducing ester to form a resulting beverage.

A stream 50 of the resulting beverage is then pumped into a heat exchanger 52 in fluid connection with blending tank 48. The resulting beverage is heated to a temperature above the melting point of the cholesterol reducing ester in heat exchanger 52. A stream 54 of the resulting beverage is then pumped into a homogenizer 56 to reduce the particle size of the cholesterol reducing ester to attain the desired mouth feel and to uniformly disperse the cholesterol reducing agent in the resulting beverage. A stream 58 of the resulting beverage is subsequently pumped into a heat exchanger 60 to heat the resulting beverage to a pasteurization temperature. The resulting beverage is held in heat exchanger 60 for less than about 60 seconds to reduce the microbial load. Immediately upon completing the pasteurization process, a stream 62 of the resulting beverage is pumped into a chiller 64 to cool the resulting beverage to a temperature below the melting point of the cholesterol reducing ester, preferably less than about 4°C. Finally, a stream 66 of the chilled resulting beverage is pumped into a packaging filler 68 in fluid connection with chiller 64 to be clean filled into containers.

Figure 2 is a schematic design illustrating another system in accordance with the present invention for making a sterol fortified beverage. A system 100 is provided to manufacture a sterol or stanol ester fortified beverage. A suitable container 102 is used to supply a stream 104 of the aqueous liquid beverage component into a heat exchanger 106 to heat the aqueous liquid beverage component to a temperature above the melting point of the cholesterol reducing ester. A suitable container 108 is used to supply a stream 110 of the cholesterol reducing ester into a melting tank 112 where the cholesterol reducing ester is melted to form a liquefied cholesterol reducing ester. A stream 16 of the heated aqueous liquid beverage component is combined with a stream 114 of the liquefied cholesterol reducing ester at a point 118 to form a concentrated mixture. A stream 120 of the concentrated mixture is pumped into homogenizer 36. The process downstream of stream 120 in Figure 2 is the same as the process described for the schematic of Figure 1 starting at homogenizer 36 in Figure 1.

## Examples

**Example #1** 48,300 grams batch of 1.0 gram of stanol ester per 240ml; two step homogenization process using a semi-solid sterol ester

First, the stanol ester and the orange juice were heated separately to 45°C. Then, 307 grams of stanol ester were blended with 48,000 grams of juice to form a mixture containing 0.64% of stanol ester by weight mixture. Next, the heated mixture was homogenized at 173 bar (2509 psi) to form a beverage. Then, the homogenized beverage was cooled to 4°C. Prior packaging the beverage, the beverage was reheated to 60°C, homogenized at 69 bar (1000 psi), heated to 96°C and held at 96°C to reduce microbial load and complete the pasteurization, and immediately chilled to 4°C to form a finished beverage. The finished beverage was a stable, uniformly-dispersed stanol ester fortified juice ready to be clean filled into containers.

**Example #2** 10,000 grams batch of 1.5 grams of stanol ester per 250ml; two step homogenization process using a semi-solid sterol ester

First, the stanol ester and the orange juice can be heated to 40°C. Then, 95.7 grams of stanol ester can be blended with 9904.3 grams of juice to form a mixture containing 0.957% of stanol ester by weight mixture. Next, the heated mixture can be homogenized at 175 bar (2538 psi) to form a beverage. Then, the beverage can first be heated to 100°C and then chilled to 4°C. Prior packaging the beverage, the beverage can be reheated to 60°C, homogenized at 175 bar (2538 psi), heated to 92°C and held at 92°C to reduce microbial load and complete the pasteurization, and cooled immediately to 4°C to form a finished beverage. The finished beverage is a stable, uniformly-dispersed stanol ester fortified juice ready to be clean filled into containers.

**Example #3** 10,000 grams batch of 0.8 grams of stanol ester per 240ml; one step homogenization process using a semi-solid sterol ester

First, the stanol ester and the orange juice can be heated to 60°C. Then, 53.2 grams of stanol ester can be blended with 9946.8 grams of juice to form a mixture containing 0.532% of stanol ester by weight mixture. Next, the mixture can be homogenized at 175 bar (2538 psi), heated to 92°C and held at 92°C to reduce microbial load and complete the pasteurization, and cooled immediately to 4°C to form a finished beverage. The finished beverage is a fortified juice ready to be clean filled into containers.

**Example #4** 75,800 grams batch of 1.0 gram of stanol ester per 240ml; two stage homogenization process; first blending step at 2X concentration using a semi-solid sterol ester

First, the stanol ester and the orange juice were heated separately to approximately 45°C. Then, 505 grams of stanol ester were blended with 39,500 grams of juice to form a concentrated mixture. The concentration of the plant stanol ester in the concentrated mixture was twice the final concentration in the juice. The temperature of the combined ingredients was 45.6°C. Next, the 2X concentration mixture was first heated to 60°C and then homogenized to 173 bar (2509 psi). Then, the homogenized concentrated mixture was chilled to 4°C. Prior to pasteurizing and packaging the beverage, 35,800 grams of the concentrated mixture were diluted with 35,800 grams of juice to form a beverage. Then, the beverage was heated to 60°C, homogenized at 173 bar (2509 psi), heated to 96°C and held at 96°C to reduce microbial load and complete the pasteurization, and cooled immediately to 4°C to form a finished beverage. The finished beverage was a stable, uniformly-dispersed stanol ester fortified juice ready to be clean filled into containers.

**Example #5** 10,000 grams batch of 0.8 grams of stanol ester per 240ml; two step homogenization process using a liquid sterol ester

First, the orange juice can be heated to 40°C. Then, 53.2 grams of liquid stanol ester can be blended with 9946.8 grams of juice to form a mixture containing 0.532% of stanol ester by weight mixture. Next, the heated mixture can first be homogenized at 175 bar (2538 psi) and then be chilled to 4°C to form a beverage. Prior packaging the beverage, the beverage can be reheated to 60°C, homogenized at 50 bar (725 psi), and heated to 92°C and held at 92°C to reduce microbial load and complete the pasteurization, and cooled immediately to 4°C to form a finished beverage. The finished beverage is a stable, uniformly-dispersed stanol ester fortified juice ready to be clean filled into containers.

**Example #6** 10,000 grams batch of 0.8 grams of stanol ester per 240ml; two step homogenization process using a powder sterol ester

First, 53.2 grams powder stanol ester can be dispersed with 9946.8 grams of orange juice to form a mixture containing 0.532% of stanol ester by weight mixture. Then, the mixture can be homogenized at 175 bar (2538 psi) to form a beverage. Prior to packaging the beverage, the beverage can be heated to 92°C and held at 92°C to reduce microbial load and complete the pasteurization, and cooled immediately to 4°C to form a finished beverage. The finished beverage is a stable, uniformly-dispersed stanol ester fortified juice ready to be clean filled into containers.

While the invention has been described with respect to certain preferred embodiments, as will be appreciated by those skilled in the art, it is to be understood that the invention is capable of numerous changes, modifications and rearrangements and such changes, modifications and rearrangements are intended to be covered by the following claims. According to one aspect of the invention, the beverage is substantially free of added emulsifier. By way of example, to the extent that any emulsifier is present, this is endogenous to the 'aqueous medium' (eg. the juice) component of the beverage. In one embodiment, the beverage contains less than 10g of emulsifier per litre, preferably less than 5g per litre, and more preferably less than 2g per litre. In another embodiment, the beverage contains less than 1g of emulsifier per litre, preferably less than 500mg per litre, and more preferably less than 200mg per litre.

**Claims**

1. A beverage comprising:

   an aqueous medium,
   a cholesterol reducing ester selected from the group consisting of sterol esters, stanol esters, and mixtures thereof; and
   wherein the sterol or stanol portion of the molecule is present in the beverage in a concentration of from about 0.8 grams/litre to about 50 grams/litre of beverage and the cholesterol reducing ester is uniformly dispersed in the aqueous.

2. The beverage of Claim 1 wherein said aqueous medium is selected from the group consisting of juices, teas, coffees, diary drinks, non-alcoholic drinks, carbonated drinks, noncarbonated drinks, and mixtures thereof.

3. The beverage of Claim 1 or Claim 2 wherein said juice is selected from the group consisting of orange, grapefruit, lime, pineapple, mango, banana, peach, grape, kiwi, cherry, watermelon, apple, strawberry, raspberry, cranberry, blueberry, blackberry, plum, passion fruit, tangerine, pomegranate, mandarin, pear, plum, mirabelle, honeydew, cantaloupe, papaya, tomato, guava, celery, beets, parsley, lettuce, spinach, carrot, and mixtures thereof.

4. The beverage of any preceding claim wherein the sterol or stanol portion of the molecule concentration is from about 1.6 grams/litre to about 12.5 grams/litre of beverage, preferably wherein the sterol or stanol portion of the molecule concentration is from about 3.2 grams/litre to about 7 grams/litre of beverage.

**5.** A method of preparing a beverage comprising:

blending a cholesterol reducing ester selected from the group consisting of sterol esters, stanol esters, and mixtures thereof and an aqueous liquid beverage component to form a mixture;

a first homogenizing of said mixture to form a resulting beverage **characterised by** a uniform dispersion of the cholesterol reducing ester in the aqueous medium; after said first homogenizing, optionally heating said resulting beverage to a pasteurization temperature;

cooling said resulting beverage to a temperature below the melting point of the cholesterol reducing ester present in the beverage; and

wherein the sterol or stanol portion of the molecule is present in the beverage in a concentration of from about 0.8 grams/litre to about 200 grams/litre of beverage.

**6.** The method of Claim 5 wherein said homogenizing is performed in the absence of an added emulsifier in the beverage composition.

**7.** The method of Claim 5 or Claim 6 wherein said blending is carried out at a temperature below the melting point of the cholesterol reducing ester, and the cholesterol reducing ester is in powder form prior to said blending.

**8.** The method of any of Claims 5-7 wherein prior to said blending said cholesterol reducing ester is first heated to a temperature above its melting point to form a liquid and said aqueous liquid beverage component is preheated to a temperature above the melting point of the cholesterol reducing ester.

**9.** The method of Claim 8 wherein the melting point of said cholesterol reducing ester is from about 35°C to about 80°C.

**10.** The method of any of Claims 5-9 wherein the concentration of the sterol or stanol portion of the molecule is from about 1.6 grams/litre to about 12.5 grams/litre of beverage.

**11.** The method of any of Claims 5-10 further comprising diluting said resulting beverage with an aqueous medium to a concentration of the sterol or stanol portion of the molecule of from about 0.8 grams/litre to about 50 grams/litre, preferably from about 1.6 grams/litre to about 12.5 grams/litre, at a diluting temperature of less than about 35°C.

**12.** The method of any of Claims 5-11 wherein the pasteurization temperature is from about 70°C to about 130°C.

**13.** The method of any of Claims 5-12 wherein said cooling is carried out at a temperature less than about 35°C.

**14.** The method of any of Claims 5-13 further comprising:

a second homogenizing of the resulting beverage to provide a uniform dispersion of the cholesterol reducing ester in the aqueous medium;

heating the homogenized resulting beverage to the pasteurization temperature for less than about 240 seconds; and

cooling the homogenized resulting beverage to a temperature below the melting point of the cholesterol reducing ester present in the beverage to form a finished beverage.

**15.** The method of Claim 14 further comprising heating the resulting beverage prior to said second homogenizing to a temperature above the melting point of the cholesterol reducing ester.

**16.** The method of any of Claims 5-15 wherein said homogenizing is performed at a pressure from about 500 psi to about 9000 psi.

**17.** A system for making a cholesterol reducing ester fortified beverage comprising:

a source of a cholesterol reducing ester selected from the group consisting of sterol esters, stanol esters, and mixtures thereof;

a source of an aqueous liquid beverage component;

a mixing device for blending the cholesterol reducing ester with the aqueous liquid beverage component to form a resulting mixture;

a homogenizer in fluid connection with the mixing device for homogenizing the resulting mixture to form a resulting beverage **characterised by** a uniform dispersion of the cholesterol reducing ester; and
a chilling device to cool the resulting beverage to a temperature below the melting point of the cholesterol reducing ester present in the beverage.

**18.** The system of Claim 17 further comprising a pasteurization device for pasteurizing the resulting beverage in fluid connection with said homogenizer to produce a pasteurized beverage wherein said pasteurization device is upstream of said chilling device.

**19.** Use of a beverage according to any of Claims 1-4, or a beverage obtainable by a method according to any of Claims 5-16;
for the manufacture of a medicament for lowering (blood) cholesterol levels in a subject, preferably in a mammal, more preferably in a human.

Figure 1

Figure 2

**EP 1 752 151 A1**

## EUROPEAN SEARCH REPORT

European Patent Office

Application Number

EP 06 25 4034

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 986 962 A (MCNEIL-PPC, INC) 22 March 2000 (2000-03-22) * [0004]; example 1 * | 1,19 | INV. A61K31/56 C07J9/00 A23L1/30 A23L2/00 A23L2/02 |
| X | US 6 200 624 B1 (MAZER TERRENCE BRUCE ET AL) 13 March 2001 (2001-03-13) * figures 1 and 2 * | 17,18 | |
| X | EP 1 298 194 A (UNILEVER N.V; UNILEVER PLC) 2 April 2003 (2003-04-02) * [0001]; examples 1a and 4a * | 1,19 | |
| X | EP 1 212 945 A (MCNEIL-PPC, INC) 12 June 2002 (2002-06-12) * [0006]; [0014]-[0020]; examples 1-3; claims 1, 2 and 6 * | 1,2,5, 13,16,19 | |
| X | US 6 576 285 B1 (BADER PRIMO ET AL) 10 June 2003 (2003-06-10) * claims 1, 4-6, 11, 12, 25, 32, 33, 35 and 37 * | 1,5,11, 19 | TECHNICAL FIELDS SEARCHED (IPC) |
| X | US 2005/051419 A1 (ZIMA GEORGE CHESTER ET AL) 10 March 2005 (2005-03-10) * example 1 * | 17 | A61K C07J A23L |
| X | WO 2004/093571 A (RAISIO BENECOL OY; POURU, ANNIINA; KUUSISTO, PAEIVI; LAHTINEN, RITVA;) 4 November 2004 (2004-11-04) * page 4, line 30 - page 5, line 12; examples 2-11; claims 1-34 * | 1-3,19 | |

The present search report has been drawn up for all claims

| Place of search | Date of completion of the search | Examiner |
|---|---|---|
| Munich | 25 September 2006 | GEORGOPOULOS, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

13

## ANNEX TO THE EUROPEAN SEARCH REPORT
## ON EUROPEAN PATENT APPLICATION NO.

EP 06 25 4034

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2006

| Patent document cited in search report | | | Publication date | Patent family member(s) | | | Publication date |
|---|---|---|---|---|---|---|---|
| EP 0986962 | A | | 22-03-2000 | AT | 311118 | T | 15-12-2005 |
| | | | | AU | 4463699 | A | 16-03-2000 |
| | | | | BR | 9903979 | A | 05-09-2000 |
| | | | | CA | 2281128 | A1 | 29-02-2000 |
| | | | | DE | 69928623 | D1 | 05-01-2006 |
| | | | | DE | 69928623 | T2 | 27-07-2006 |
| | | | | DK | 0986962 | T3 | 02-01-2006 |
| | | | | ES | 2253864 | T3 | 01-06-2006 |
| | | | | JP | 2000102361 | A | 11-04-2000 |
| | | | | NO | 994195 | A | 01-03-2000 |
| | | | | NZ | 337359 | A | 30-05-2003 |
| | | | | US | 6123978 | A | 26-09-2000 |
| | | | | US | 6399137 | B1 | 04-06-2002 |
| US 6200624 | B1 | | 13-03-2001 | AU | 720158 | B2 | 25-05-2000 |
| | | | | AU | 1838297 | A | 20-08-1997 |
| | | | | CA | 2247368 | A1 | 31-07-1997 |
| | | | | CN | 1214616 | A | 21-04-1999 |
| | | | | CZ | 9802364 | A3 | 17-02-1999 |
| | | | | EP | 0876110 | A1 | 11-11-1998 |
| | | | | HU | 9901107 | A2 | 28-07-1999 |
| | | | | IL | 125502 | A | 31-10-2000 |
| | | | | JP | 2000504221 | T | 11-04-2000 |
| | | | | NO | 983433 | A | 28-09-1998 |
| | | | | NZ | 331084 | A | 29-11-1999 |
| | | | | WO | 9726804 | A1 | 31-07-1997 |
| EP 1298194 | A | | 02-04-2003 | EP | 0911385 | A1 | 28-04-1999 |
| EP 1212945 | A | | 12-06-2002 | AU | 9713001 | A | 13-06-2002 |
| | | | | CA | 2364726 | A1 | 07-06-2002 |
| | | | | JP | 2002204679 | A | 23-07-2002 |
| | | | | US | 2002122865 | A1 | 05-09-2002 |
| US 6576285 | B1 | | 10-06-2003 | NONE | | | |
| US 2005051419 | A1 | | 10-03-2005 | US | 2005053712 | A1 | 10-03-2005 |
| WO 2004093571 | A | | 04-11-2004 | AU | 2004231327 | A1 | 04-11-2004 |
| | | | | BR | PI0409598 | A | 02-05-2006 |
| | | | | CA | 2521768 | A1 | 04-11-2004 |
| | | | | CN | 1780561 | A | 31-05-2006 |
| | | | | EP | 1615511 | A1 | 18-01-2006 |
| | | | | MX | PA05011259 | A | 24-01-2006 |

EPO FORM P0459

For more details about this annex : see Official Journal of the European Patent Office, No. 12/82

**REFERENCES CITED IN THE DESCRIPTION**

**Patent documents cited in the description**

- US 5958913 A

- US 6174560 B